# EUROPEAN PATENT APPLICATION

(11) **EP 1 736 768 A1**
(43) Date of publication of application: **27.12.2006**
(21) Application number: 05013417.0
(22) Date of filing: 22.06.2005
(51) Int. Cl.: G01N 33/00, G01N 27/414, G01N 27/18, G01N 25/48

(54) **Device and method for gas sensing**

(71) Applicant: AppliedSensor Sweden AB, 583 30 Linköping (SE)
(72) Inventor: Löfdahl, Mikael, 58593 Linköping (SE); Mitrovics, Jan Levente Walther Tibor, 72076 Tubingen (DE)
(74) Representative: Willquist, Bo Lorentz

(57) **Abstract**

A gas sensing method using a sensing device (10) comprising a field-effect gas sensor (11; 20) adapted to provide a signal indicative of the gas concentration. The method comprises the steps of heating (40) a part (13; 22) of the sensing device (10), detecting (41) a heat transfer from the heated part (13; 22), and determining (42) the gas concentration using the detected heat transfer and the signal from the field-effect gas sensor (11; 20).

Through this method, it is possible to obtain a correct and reliable gas concentration reading over an extended concentration range. Furthermore, the start-up time of a sensing device can be shortened.

## Description

### Technical Field of the Invention

The present invention relates to a method, for detecting and measuring a gas concentration using a sensing device comprising a field-effect gas sensor adapted to provide a signal indicative of the gas concentration.

The invention further relates to a sensing device, for detecting and measuring a gas concentration, comprising a field-effect gas sensor adapted to provide a signal indicative of the gas concentration.

### Technical Background

Gas sensors have found numerous uses as detectors or analytical instrumentation for detecting the presence of, for example, hazardous or toxic gases. To cater for the varying requirements of different applications as well as of the particular gas to be sensed, a number of sensing methods and devices have been developed in the past. These different methods have strengths and weaknesses making them more or less suited for certain applications.

As a concrete example of an application which has led the development towards more compact, inexpensive and low-power gas sensors, the automotive industry could be mentioned. Due to the expected increase in the number of hydrogen powered cars - by fuel cells or other means - there is a demand for hydrogen sensors in particular which fulfil the requirements of the automotive industry.

A candidate for use as a gas sensor in future hydrogen-powered cars is the field-effect gas sensor. Field-effect gas sensors are based on Metal Oxide Semiconductor Field Effect Transistors (MOSFETs) with a gate usually consisting of a catalytic metal. Field-effect gas sensors are commonly operated at elevated temperatures (typically 140°C) and are therefore heated. Which gas is detected by the field-effect sensor depends on which material is chosen as a gate, the catalytic metal properties and the operating temperature of the device.

In order to make the field-effect gas sensor suitable for applications in the automotive industry or for handheld devices and so on, development has been carried out in order to reduce the power consumption of field-effect sensors.

In WO 00/75619, a micro-machined field-effect gas sensor is disclosed. Due to the low thermal mass, small size and good thermal isolation of the disclosed device, the power consumption of the gas sensor could be reduced.

Even though this field-effect gas sensor through its lowered power consumption fulfils several of the requirements for gas sensors to be used in, for example, the automotive industry, there is still room for improvement. Particularly, there is a need for a gas sensing device with an even wider detection range.

Furthermore, there is a need for a gas sensing device that is even more suitable for safety-related applications.

### Objects of the Invention

In view of the above-mentioned and other drawbacks of the prior art, a general object of the present invention is to provide an improved gas sensing device and method.

A further object of the present invention is to enable gas detection over a wider range.

An additional object of the present invention is to provide a gas sensing device that is even more suitable for safety-related applications.

### Summary of the Invention

According to a first aspect of the invention, these and other objects are achieved through a method, for detecting and measuring a gas concentration using a sensing device comprising a field-effect gas sensor adapted to provide a signal indicative of the gas concentration, comprising the steps of heating a part of the sensing device, detecting a heat transfer from the heated part of the sensing device, the heat transfer being related to said gas concentration, and determining the gas concentration using the detected heat transfer and the signal from the field-effect gas sensor.

According to a second aspect of the invention, these and other objects are achieved by a sensing device, for detecting and measuring a gas concentration, comprising a field-effect gas sensor adapted to provide a signal indicative of the gas concentration, the sensing device further comprising a heater for heating a part of the sensing device, detecting means for detecting a heat transfer from the heated part of the sensing device, the heat transfer being related to the gas concentration, and processing means for determining the gas concentration using the detected heat transfer and the signal from the field-effect gas sensor.

By "sensing device" should here be understood a single sensor or a number of sensing units arranged to enable the combination of signals from these sensing units.

The part of the sensing device that is heated can be heated by an external heater or, preferably, by a heater provided on the sensing device. The latter heater could, for example, be a micro-hotplate heater, which could comprise a resistive heater or an active element, such as a transistor, preferably an FET.

Field-effect gas sensors are based on Metal Oxide Semiconductor Field Effect Transistors (MOSFETs) with a gate of a catalytic metal, such as Pt, Pd, Ir. The gate is the active part of the field-effect sensor and as gas molecules interact with the gate, the transistor characteristics shift. Normally, a field-effect gas sensor is fed by a constant current source and the output of the sensor is a voltage which is approximately proportional to the logarithm of the gas concentration in the ambient. Field-effect gas sensors usually operate at elevated temperatures (typically 140°C) and are therefore heated. Which gas is detected by the field-effect sensor depends on which material is chosen as a gate layer and the operating temperature of the device.

Field-effect gas sensors are, in general, able to measure very low concentrations of a gas. At higher concentrations field-effect gas sensors saturate. On the other hand, field-effect sensors can be made very selective to a particular gas to be sensed or measured.

The present invention is based on the realisation that a concentration of a gas can be measured more accurately and over a wider range by using a signal indicative of a gas concentration obtained from a field-effect gas sensor together with a detected heat transfer. This signal from the field-effect sensor and detected heat transfer can be processed by processing means in the form of, for example, an integrated or external processor or a computer, the output from the processing means typically being a gas concentration value or a measure indicative thereof. The output could also be a warning when a certain gas concentration threshold value has been exceeded.

The heat transfer from a heated body in general and the gas sensing device according to the present invention in particular, mainly depends on convection properties such as heat conductivity, viscosity and density of the investigated gas and also of the geometry of the sensor system.

Through a detection of the heat transfer, a prediction of the gas concentration can be obtained before the output of the field-effect gas sensor has reached a stable value. The start-up time of the sensing device is thus shortened. The shortened start-up time enables the use of pulsed operation with a lower duty cycle compared to prior art. Thereby, an even lower power operation may be obtained.

Through a detection of the heat transfer, it is further usually possible to obtain gas concentration readings over a wide range. For hydrogen gas in nitrogen gas mixtures, for example, hydrogen gas concentration readings from around 1 percent to 100 percent are typically obtainable.

In general, different gases have different thermal conductivities and can therefore be distinguished from each other through detection of the heat transfer from a heated element. However, some interesting gases exhibit thermal conductivities sufficiently close to each other to potentially give rise to erroneous measurements. Examples of such gases are hydrogen and helium.

Through the method and device according to the present invention, it is thus possible to obtain a correct gas concentration reading over an extended concentration range.

Furthermore, the method and device according to the invention enables to reliably check whether the measured gas is the intended target gas. Thereby, false alarm events can be avoided.

Furthermore, for some gases, the response time of the sensing device can be shortened compared to field-effect gas sensors.

Additionally, improved safety and reliability can be obtained through the use of two complementary sensing principles. The sensing device according to the present invention is therefore particularly useful for applications in which safety is an aspect.

The method and device according to the present invention furthermore enables self-calibration of the gas sensing device. For example, a detected heat transfer indicating that no target gas is present may be used to zero the field-effect gas sensor and vice versa.

Preferably, the step of detecting the heat transfer from the heated part of the sensing device can comprise measuring the temperature of the heated part of the sensing device.

For example, a heated structure in the sensing device can be positioned at some distance from a reference structure and the heat conductivity through the gas to be investigated from the heated structure to the reference structure can be detected by measuring the temperature of this heated structure. The heat transfer detection could be refined further by, in addition, measuring the temperature of the reference structure.

It would also be possible to detect the heat transfer from a heated structure to the surrounding gas without the provision of a reference structure. The resulting temperature of the heated structure and the surrounding gas would then depend on the supplied power and the heat capacity of the gas.

The temperature of selected parts of the sensing device and/or the ambient can be measured in a number of ways, such as by an external probe - IR or similar - or using a temperature sensor provided on the sensing device. In analogy with the previously mentioned heater, such a temperature sensor could be provided in a number of ways, such as through resistive and/or active structures. Such resistive structures could, for example, comprise Al, poly-Si and/or n-Si diffusion resistors and active structures could include transistors and/or diodes. A thermopile configuration could also be used to measure the temperature. Of course, a plurality of sensors could be arranged on the sensing device in order to measure temperature in different positions and thereby gaining additional information regarding the temperature distribution and/or a more precise temperature reading at certain locations on the sensing device. Temperature sensors could also be integrated in the field-effect sensor or, alternatively, the temperature dependence of the electrical characteristics of the field-effect sensor could be used to measure the temperature.

According to one embodiment of the present invention, the step of detecting the heat transfer from the heated part of the sensing device can further comprise regulating the temperature of the heated part, and detecting an electrical power supplied to the sensing device, the supplied power being indicative of the heat transfer.

By regulating the temperature and detecting the electrical power supplied, the heating power dissipation can be detected and, via the regulated temperature, correlated to the heat transfer from the sensing device.

The supplied power is mainly constituted by the electrical power supplied to the heater which is arranged to heat a part of the sensing device. Consequently, the power supplied to the heater in order to heat that part of the sensing device is normally the only power detected. However, also the current supplied to other parts of the sensing device, such as other sensors and/or processors may contribute to the heating power. In some cases it may be necessary to take this additional power into consideration when determining the heat transfer from the sensing device.

Advantageously, the temperature of the heated part of the sensing device may be regulated to a constant value.

By regulating the temperature of a part of the sensing device to a constant value, the supplied heating power - possibly including part of the previously mentioned driving power - is a direct measure of the heat transferred from the heated part of the sensing device to the ambient. The amount of heat thus transferred is an indication of the gas concentration.

According to another embodiment of the invention, the step of detecting the heat transfer from the heated part of the sensing device can comprise measuring the decreasing temperature of the heated part of the sensing device after termination of the heating step.

After heating the part to be heated to a predetermined (suitable) temperature, which may be dependent on the gas to be detected, the heated part can be allowed to cool down. By measuring the temperature decrease after the termination of heating, a measure indicative of the heat transfer from the heated part can be obtained.

An effect of this type of measurement is that interference resulting from noise generated by an internal or external heater can be eliminated or reduced.

According to a further embodiment of the invention, the step of detecting the heat transfer from the heated part of the sensing device can comprise measuring the increasing temperature of the heated part during heating.

Through this method a shortened response or sensor start-up time can be achieved and a fast indication of the presence of a, potentially harmful, gas thereby obtained. This indication may lead to the provision of a warning signal.

According to yet another embodiment of the present invention, the heater or/and temperature sensor can be part(s) of the field-effect gas sensor.

By providing the above-described heater or/and temperature sensor as integral parts of the field-effect gas sensor, the field-effect gas sensor can additionally be used for heat transfer detection. Thereby, a compact and potentially low-cost gas sensor with much improved sensing range and response time can be realised.

An advantageous way of realising a sensing device according to the present invention is through micromachining, whereby at least a part of the sensing device would be a so-called MEMS (Micro ElectroMechanical System).

Through the use of micromachining, very small structures can be realised by more-or-less standard semiconductor batch manufacturing processes. The infrastructure of modern semiconductor fabs can be used. For example, standard CMOS processes are useful and advantageous from a cost point-of-view. Through micromachining the manufacturing of low-power, high integration gas sensors is facilitated.

### Brief Description of the Drawings

These and other aspects of the present invention will now be described in more detail, with reference to the appended drawings showing currently preferred embodiments of the invention.

Fig. 1 is an illustration of an example of an application for a gas sensing device according to the present invention.

Fig. 2a is a schematic view of a sensing device according to a first embodiment of the second aspect of the present invention.

Fig. 2b is a schematic view of a part of a sensing device according to a second embodiment of the second aspect of the present invention.

Fig. 3a is a schematic view of first configuration for mounting a sensing device or a part of a sensing device on a board.

Fig. 3b is a schematic view of a second configuration for mounting a sensing device or a part of a sensing device on a board.

Fig. 4 is a flow chart illustrating a method, for detecting and measuring a gas concentration, according to a first embodiment of the first aspect of the present invention.

Fig. 5a is a flow chart illustrating a method, for detecting and measuring a gas concentration, according to a second embodiment of the first aspect of the present invention.

Fig. 5b is a schematic diagram illustrating the method in fig. 5a.

Fig. 6a is a flow chart illustrating a method, for detecting and measuring a gas concentration, according to a third embodiment of the first aspect of the present invention.

Fig. 6b is a schematic diagram illustrating the method in fig. 6a.

Fig. 7a is a flow chart illustrating a method, for detecting and measuring a gas concentration, according to a fourth embodiment of the first aspect of the present invention.

Fig. 7b is a schematic diagram illustrating the method in fig. 7a.

### Detailed Description of Preferred Embodiments of the Invention

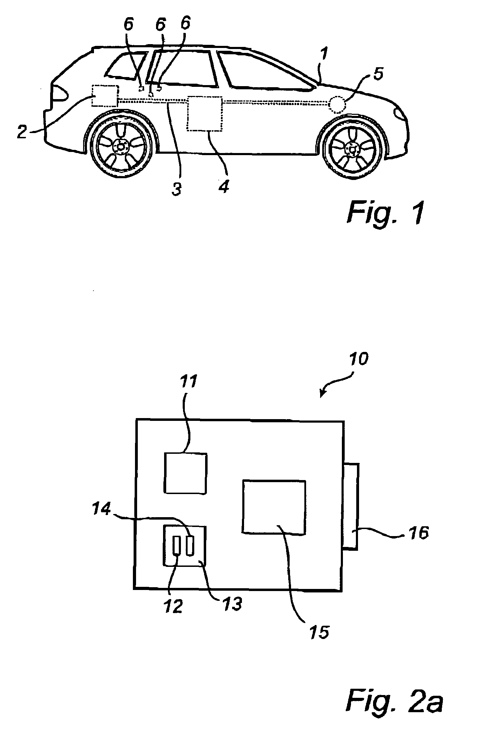

In fig. 1, an example of an application for a gas sensing device according to the present invention is shown. Here, a schematic fuel-cell powered car 1 is provided with a fuel tank 2, containing hydrogen gas, a supply line 3, leading gas to a fuel cell 4, where the hydrogen gas is used to generate an electric current, which is used to power the engine 5. In order to guarantee the safety of the people travelling in the car 1 as well as others nearby, one or several gas sensors 6 are positioned on suitable locations inside the car. These gas sensors 6, which are expected to number approximately 10-20 per vehicle, should be specific to hydrogen gas, have a short response time and be able to cover a wide concentration range.

Fig. 2a schematically shows a first embodiment of a sensing device 10 according to the present invention.

The sensing device 10 comprises a field-effect gas sensor 11, a heater 12 for heating a part of the sensing device 10, here in the form of a separate heat transfer sensing unit 13, detecting means in the form of a temperature sensor 14, a processor 15 and an interface 16.

This sensing device 10 is an example of a gas sensor which would have suitable properties for the application illustrated in fig. 1, since the field-effect gas sensor 11 and the heat transfer sensing unit 13 have complementary strengths, which combined contribute to gas-specific sensing, short response time and wide detection range.

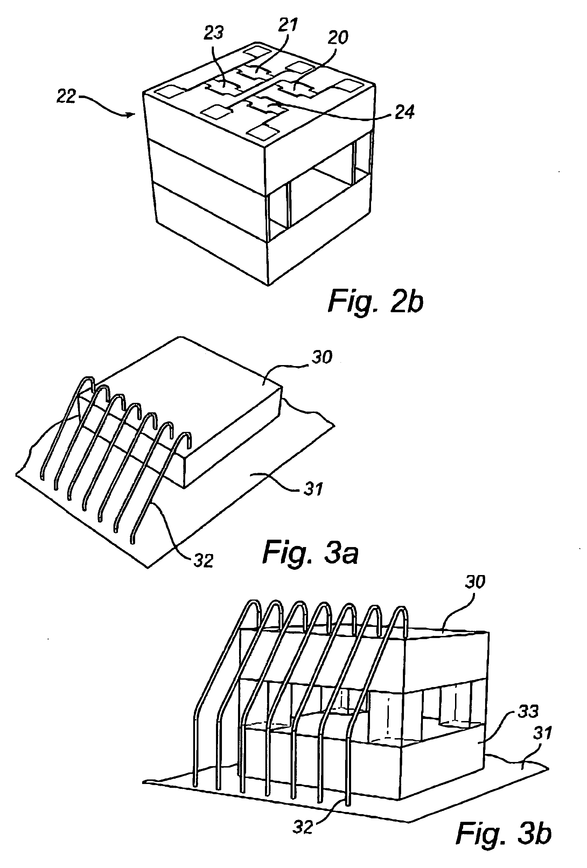

In fig. 2b, a part of a second embodiment of a gas sensing device according the present invention is schematically shown. Here, the field-effect gas sensor 20 and the heat transfer sensing unit 21 are provided in the same sensing unit 22. The field-effect gas sensor 20 is heated to its operational temperature by a heater 23. The temperature of the field-effect gas sensor is measured by a temperature sensor 24 and the heat transfer from the sensing unit 22 is detected. Signals from the field-effect sensor 20 as well as information regarding the heat transfer are collected by a processing unit which, in this example, is provided outside the sensing unit 22 and not included in fig 2b.

Above, two embodiments of gas sensing devices according to the present invention have been described. As would be understood by a person skilled in the art, several other variations are also within the scope of the invention. For example, a field-effect sensing unit and a heat transfer sensing unit could be provided as physically separated units and not be mounted on the same circuit board as long as both of these units are configured to be connected to the same processing unit.

Furthermore, the field-effect gas sensor, the heat transfer sensing unit and the processing unit could be integrated on the same chip, forming a very compact system-on-chip.

In fig. 3a, a first example of a mounting configuration of the sensing device according to the second aspect of the invention is shown. According to this example, the sensing device 30, or part of the sensing device, is suspended at a distance from a circuit board 31 by bond wires 32.

In fig. 3b, a second example of a mounting configuration is shown. Here, the sensing device 30 is mounted on the circuit board 31 via a glass carrier 32, in order to thermally insulate the sensing device 30 from the circuit board 31.

The purpose of the above described mounting configurations is to thermally insulate the sensing element 30 from the heat sink constituted by the circuit board 31.

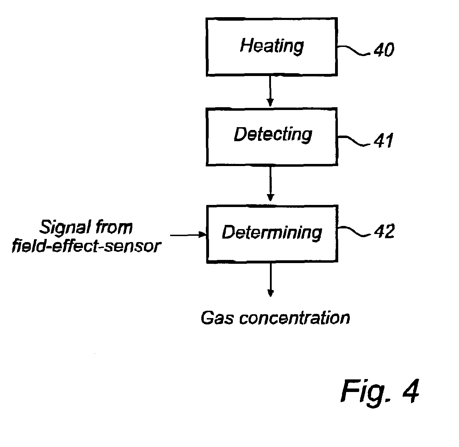

In fig. 4, a flow-chart illustrating a first embodiment of a gas sensing method according to the present invention is shown. With reference to the sensing device in fig 2a, the method according to this embodiment comprises the steps of heating 40 a part of the sensing device 10, detecting 41 a heat transfer from the heated part 13 of the sensing device and determining 42 the gas concentration using the detected heat transfer and a signal from the field-effect gas sensor 11. The above-described method is by no means limited to the sensing device in fig 2a.

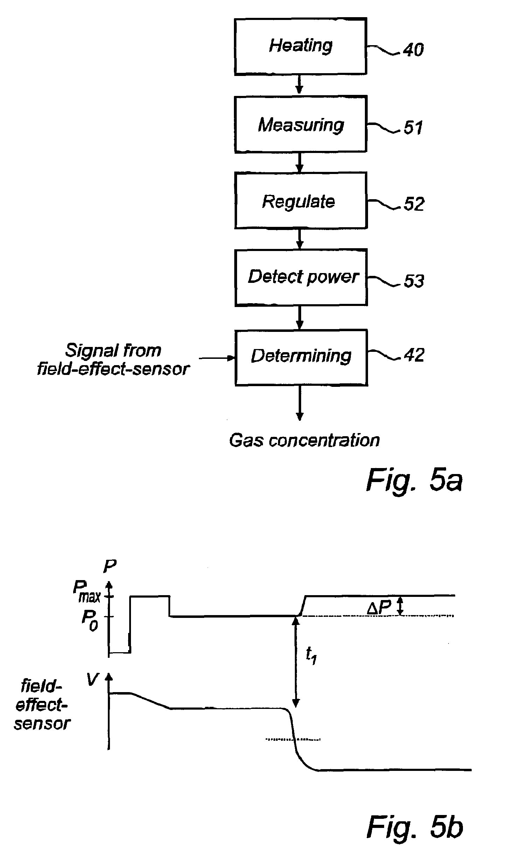

In fig. 5a, a second embodiment of the method according to the invention is shown. According to this embodiment, the step 41 of detecting a heat transfer from the heated part 13 of the sensing device 10 includes the steps of measuring 51 the temperature of the heated part 13 of the sensing device 10, regulating 52 the temperature of the heated part 13 to a constant value and detecting 53 the heating power supplied to the heater 12 used to heat the heated part 13 of the sensing device 10. The above-described method is by no means limited to the sensing device in fig 2a.

Through the detection 53 of this heating power, the gas concentration can be determined in a way illustrated in fig. 5b and here exemplified using the embodiment of the sensing device wherein the field-effect gas sensor 20 and the heat transfer sensing unit 21 are integrated in the same element 22 as shown in fig 2b. Generally, the power supplied to the heater 23 is kept at a maximum value Pₘₐₓ until a suitable temperature has been reached. In this embodiment of the sensing device, the suitable temperature can be the working temperature of the field-effect gas sensor 20, T_{FE}. The temperature is then kept constant at this suitable value by controlling the electric power supplied to the heater 23. When none of the gas to be detected is present, the supplied power will be constant Po and its value will indicate a gas concentration of 0%. Upon the entry of the gas to be detected, for example hydrogen gas, into the sensor at a time t₁, more electric power will need to be supplied in order to keep the temperature at a constant value. The added electric power ΔF will be a measure of the hydrogen gas concentration. Some time after the gas-entry time t₁, the field-effect sensor 20 will indicate an increased concentration through a reduced output voltage, as is also shown in fig 5b. This signal from the field-effect sensor 20 confirms that the gas concentration indicated through detection of the heat transfer concerns the correct gas. If the gas concentration is higher than in the order of one percent, the field-effect sensor 20 may saturate.

If the gas concentration is too low to be sensed through monitoring of the supplied electric power, the field-effect sensor 20 will give a signal indicative of the gas concentration.

To be certain that the gas concentration sensed through detection 53 of the added electric power ΔP is the concentration of the gas to be sensed, in this case hydrogen gas, the signal from the field-effect sensor 20 can be used. If a signal from the heat transfer unit 21 is present and there is no signal from the field-effect sensor 20, an alarm should not be initiated since the wrong gas has been sensed.

In the event of a high concentration of gas already being present during the heating of the part of the sensing device to be heated, a signal from the field-effect sensor obtained already during warm-up to the suitable working temperature can be used to provide a quick alert to persons potentially in danger, for example inside a fuel-cell powered vehicle. The above-described method is by no means limited to the sensing device in fig 2b.

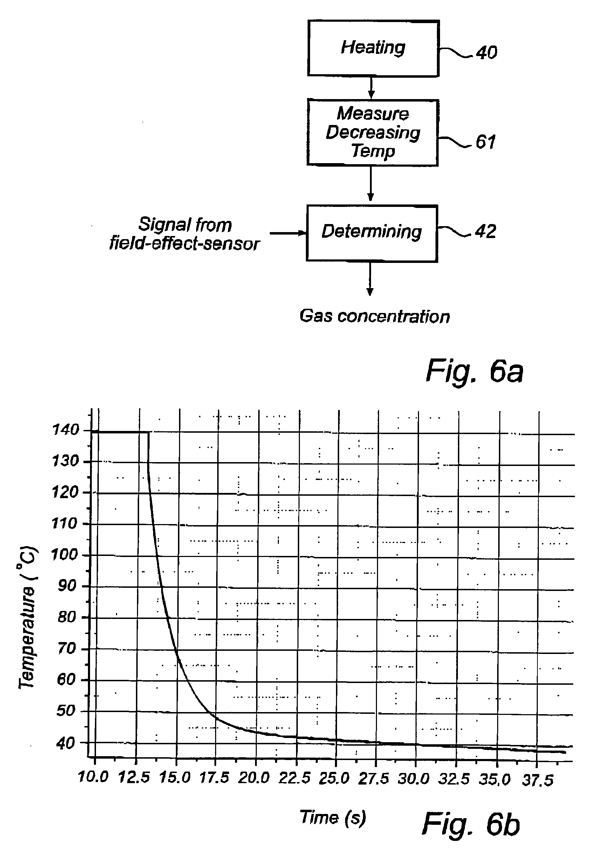

In fig. 6a, a third embodiment of the method according to the invention is shown. According to this embodiment, which is here described with reference to the sensing device in fig 2b, the step 41 of detecting a heat transfer from the heated part 22 of the sensing device includes the step 61 of measuring the decreasing temperature of the heated part 22 of the sensing device following termination of the heating.

By measuring the decreasing temperature after termination of heating, the amount of heat transferred from the heated part 22 can be measured in a way schematically illustrated by the diagram in fig. 6b. As can be seen in this diagram, the temperature decreases from, in this case, the working temperature of the field-effect sensor T_{W}. The decrease is dependent on the heat conductivity and/or the heat capacity of the surrounding gas and a measure of the gas concentration can be obtained by measuring the slope of the curve.

A reason for using this method is that the amount of noise affecting the temperature measurement is reduced by turning off the heater 23. Typically, the sensing device is here operated by cycling the temperature, the heater first being turned on to allow for a measurement by the field-effect sensor 20 and then being turned off in order for the above-described temperature measurement to take place. The above-described method is by no means limited to the sensing device in fig 2b.

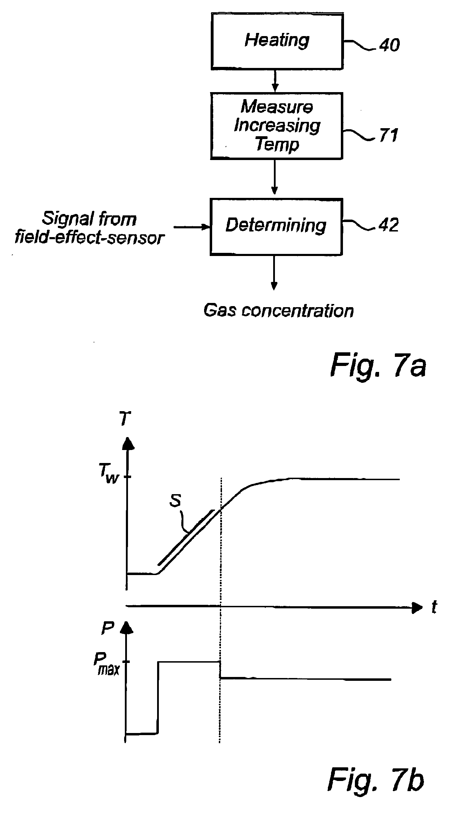

In fig. 7a, a fourth embodiment of the method according to the invention is shown. According to this embodiment, the step 41 of detecting a heat transfer from the heated part 22 of the sensing device includes the step 71 of measuring the increasing temperature of the heated part 22 of the sensing device during heating, typically during heating to the field-effect sensor 20 working temperature Tw.

By measuring the increasing temperature during heating, the amount of heat transferred from the heated part 22 can be measured in a way schematically illustrated by the diagram in fig. 7b. As can be seen in this diagram, the temperature increases towards the working temperature of the field-effect sensor T_{W}. The increase is dependent on the heat conductivity and/or the heat capacity of the surrounding gas and a measure of the gas concentration can be obtained by measuring the slope S of the curve.

## Claims

1. A method, for detecting and measuring a gas concentration using a sensing device (10) comprising a field-effect gas sensor (11; 20) adapted to provide a signal indicative of the gas concentration, comprising the steps of:
- heating (40) a part (13; 22) of the sensing device (10);
- detecting (41) a heat transfer from said part (13; 22) of the sensing device (10), said heat transfer being related to said gas concentration, and;
- determining (42) the gas concentration using said detected heat transfer and said signal from said field-effect gas sensor (11; 20).

2. A method according to claim 1, wherein said step of detecting (41) the heat transfer from said part (13; 22) of the sensing device (10) comprises:
- measuring (51) the temperature of said part (13; 22) of the sensing device (10).

3. A method according to claim 2, wherein said step of detecting (41) the heat transfer from said part (13; 22) of the sensing device (10) further comprises:
- regulating (52) the temperature of said part (13; 22) of the sensing device (10), and;
- detecting (53) an electrical power supplied to the sensing device (10), said supplied power being indicative of said heat transfer.

4. A method according to claim 3, wherein said temperature is regulated to a constant value.

5. A method according to claim 2, wherein said step of detecting (41) the heat transfer from said part (13; 22) of the sensing device (10) further comprises:
- measuring the decreasing temperature (61) of said part (13; 22) of the sensing device (10) after termination of the heating step (40).

6. A method according to claim 2, wherein said step of detecting (41) the heat transfer from said part (13; 22) of the sensing device (10) further comprises:
- measuring the increasing temperature (71) of said part (13; 22) of the sensing device (10) during heating.

7. A sensing device (10), for detecting and measuring a gas concentration, comprising a field-effect gas sensor (11; 20) adapted to provide a signal indicative of the gas concentration, said sensing device (10) further comprising:
- a heater (12) for heating a part of the sensing device (10);
- detecting means (14; 24) for detecting a heat transfer from said part (13; 22) of the sensing device (10), said heat transfer being related to said gas concentration, and;
- processing means (15) for determining the gas concentration using said detected heat transfer and said signal from said field-effect gas sensor (11; 20).

8. A sensing device (10) according to claim 7, wherein said detecting means comprise:
- a temperature sensor (14; 24) for measuring the temperature of said part (13; 22) of the sensing device (10).

9. A sensing device (10) according to claim 7 or 8, wherein said detecting means comprise:
- regulating means for regulating the temperature of said part (13; 22) of the sensing device (10), and;
- means for detecting an electrical power supplied to the sensing device (10), said supplied power being indicative of said heat transfer.

10. A sensing device (10) according to claim 9, wherein said regulating means are configured to regulate the temperature to a constant value.

11. A sensing device (10) according to claim 7 or 8, wherein said detecting means (14; 24) are configured to measure a decreasing temperature of said part (13; 22) of the sensing device (10) after termination of heating.

12. A sensing device (10) according to claim 8, wherein said temperature sensor (14; 24) is configured to measure an increasing temperature of said part (13; 22) of the sensing device (10) during heating.

13. A sensing device (10) according to any one of claim 7 - 12, wherein said heater (12; 23) is a part of said field-effect gas sensor (11).

14. A sensing device (10) according to any one of claim 8 - 13, wherein said temperature sensor (14; 24) is a part of said field-effect gas sensor (11).

15. A sensing device (10) according to any one of claim 7 - 14, wherein said sensing device (10) is manufactured by micromachining.
